# EUROPÄISCHE PATENTSCHRIFT

(11) **EP 0 731 077 B1**
(45) Veröffentlichungstag und Bekanntmachung des Hinweises auf die Patenterteilung: **13.10.1999**
(21) Anmeldenummer: 96103300.8
(22) Anmeldetag: 04.03.1996
(51) Int. Cl.: C07C 47/22, C07C 45/35, C07C 57/04, C07C 51/25, C07C 45/33, C07C 51/215, C07C 5/333, C07C 11/06

(54) **Verfahren zur Herstellung von Acrolein, Acrylsäure oder deren Gemisch aus Propan**
Process for the preparation of acrolein, acrylic acid or their mixtures from propane
Procédé pour la préparation d'acroléine, d'acide acrylique ou de leurs mélanges à partir de propane

(30) Priorität: 10.03.1995 DE 19508558; 13.07.1995 DE 19525504
(43) Veröffentlichungstag der Anmeldung: 11.09.1996
(73) Patentinhaber: BASF AKTIENGESELLSCHAFT, 67056 Ludwigshafen (DE)
(72) Erfinder: Hefner, Werner, Dr., 68623 Lampertheim (DE); Machhammer, Otto, Dr., 67281 Kirchheim (DE); Neumann, Hans-Peter, Dr., 67067 Ludwigshafen (DE); Tenten, Andreas, Dr., 67487 Maikammer (DE); Ruppel, Wilhelm, Dr., 67227 Frankenthal (DE); Vogel, Herbert, Dr., 67069 Ludwigshafen (DE)

(56) Entgegenhaltungen:
- EP-A- 0 117 146
- GB-A- 2 118 939

## Beschreibung

Vorliegende Erfindung betrifft ein Verfahren zur Herstellung von Acrolein, Acrylsäure oder deren Gemisch aus Propan, bei dem an
A) in einer ersten Stufe A das Propan einer partiellen heterogen katalysierten Dehydrierung in der Gasphase zu Propylen unterwirft,
B) das Propylen und nicht umgesetztes Propan enthaltende Produktgasgemisch der Stufe A in einer zweiten Stufe B zur Beschickung eines Oxidationsreaktors verwendet und in dem Oxidationsreaktor das Propylen einer selektiven heterogen katalysierten Gasphasen-Partialoxidation mit molekularem Sauerstoff zu Acrolein, Acrylsäure oder deren Gemisch als Zielprodukt unterwirft, wobei als Sauerstoffquelle reiner Sauerstoff verwendet wird, und
C) aus dem im Rahmen der partiellen Oxidation des Propylens in der Stufe B anfallenden Produktgasstrom in einer dritten Stufe C das Zielprodukt abtrennt und wenigstens das im Produktgasstrom der Stufe B enthaltene nicht umgesetzte Propan in die Dehydrierungsstufe A zurückführt.

Acrylsäure ist eine bedeutende Grundchemikalie, die unter anderem als Monomeres zur Herstellung von Polymerisaten Verwendung findet, die beispielsweise in disperser Verteilung in wäßrigem Medium befindlich als Bindemittel angewendet werden. Acrolein ist ein bedeutendes Zwischenprodukt, beispielsweise für die Herstellung von Glutardialdehyd, Methionin, Folsäure und Acrylsäure.

Es ist allgemein bekannt, Acrylsäure durch heterogen katalysierte Gasphasenoxidation von Propylen mit molekularem Sauerstoff an im festen Aggregatzustand befindlichen Katalysatoren herzustellen (vgl. z.B. DE-A 19 62 431, DE-A 29 43 707, DE-PS 1 205 502, EP-A 257 565, EP-A 253 409, DE-AS 22 51 364, EP-A 117 146, GB-PS 1 450 986 und EP-A 293 224).

Bei den zu verwendenden Katalysatoren handelt es sich normalerweise um Oxidmassen. Die katalytisch aktive Oxidmasse kann neben Sauerstoff lediglich ein anderes Element oder mehr als ein anderes Element (Multielementoxidmassen) enthalten. Besonders häufig kommen als katalytisch aktive Oxidmassen solche zur Anwendung, die mehr als ein metallisches, insbesondere übergangsmetallisches, Element umfassen. In diesem Fall spricht man von Multimetalloxidmassen. Üblicherweise sind die Multielementoxidmassen keine einfachen physikalischen Gemische von Oxiden der elementaren Konstituenten, sondern heterogene Gemische von komplexen Polyverbindungen dieser Elemente.

In der Regel erfolgt die heterogen katalysierte Gasphasenoxidation von Propylen zu Acrylsäure bei erhöhter Temperatur (normalerweise einige hundert °C, typischerweise 200 bis 450°C).

Da die heterogen katalysierte Gasphasenoxidation von Propylen zu Acrylsäure stark exotherm verläuft, führt man sie in zweckmäßiger Weise im Wirbelbett oder in Vielkontaktrohr-Festbettreaktoren durch, durch deren die Kontaktrohre umgebenden Raum ein Wärmeaustauschmittel geleitet wird. Letztere Verfahrensweise ist die bevorzugte (vgl. z.B. DE-A 44 31 957 und DE-A 44 31 949). Der Arbeitsdruck (Absolutdruck) beträgt normalerweise 1 bis 10 bar. Die Zielumsetzung erfolgt während der Verweilzeit des Reaktionsgasgemisches in der Katalysatorbeschickung, durch die es geleitet wird.

Wie dem Fachmann allgemein bekannt ist, läuft die heterogen katalysierte Gasphasen-Partialoxidation von Propylen zu Acrylsäure prinzipiell in zwei längs der Reaktionskoordinate aufeinanderfolgenden Schritten ab, von denen der erste zum Acrolein und der zweite vom Acrolein zur Acrylsäure führt. Aufgrund dieser Inhärenz schließt eine Eignung des erfindungsgemäßen Verfahrens für die gasphasenkatalytisch oxidative Herstellung von Acrylsäure aus Propylen automatisch eine Eignung für die gasphasenkatalytisch oxidative Herstellung von Acrolein aus Propylen ein, da die Acrylsäureherstellung jederzeit auf der Acroleinstufe abgebrochen werden kann.

Infolge des ausgeprägt exothermen Charakters der Partialoxidation des Propylens werden die Oxidationsreaktoren üblicherweise mit einem Gasgemisch beschickt, das die Reaktanten molekularer Sauerstoff und Propylen mit einem sich unter den Bedingungen der gasphasenkatalytischen Partialoxidation im wesentlichen inert verhaltenden Gas verdünnt enthält. Hier werden darunter Verdünnungsgase verstanden, deren Bestandteile unter den Bedingungen der heterogen katalysierten Gasphasen-Partialoxidation, jeder Bestandteil für sich betrachtet, zu mehr als 95 mol-%, vorzugsweise zu mehr als 98 mol-%, unverändert erhalten bleiben. Üblicherweise vereinigt das inerte Verdünnungsgas den größten Volumenanteil der drei Bestandteile des Beschickungsgasgemisches auf sich.

Die klassischen Verfahren der heterogen katalysierten Gasphasenoxidation von Propylen zu Acrolein und/oder Acrylsäure empfehlen Wasserdampf und/oder Stickstoff als inertes Verdünnungsgas (vgl. z.B. US-A 4,147,885, Spalte 1, Zeilen 20 bis 35, DE-A 20 56 614, Seite 2, letzte beiden Zeilen, DE-AS 20 09 172, Spalte 4, Zeilen 40 bis 45, DE-A 22 02 734, Seite 4, Zeilen 18 bis 22, DE-A 30 06 894, Seite 6, Zeile 21 und DE-A 24 36 818, Seite 2, Absatz 3, wobei die DE-A 20 56 614 die besondere Eignung von Wasserdampf als inertem Verdünnungsgas auf dessen relativ erhöhte molare Wärmekapazität zurückführt (Seite 4, Absatz 2, Zeile 4), wohingegen die DE-AS 22 51 364 bezüglich der Verwendung von Stickstoff als inertem Verdünnungsgas den Kostenaspekt (Luft als Quelle des Oxidationsmittels) anführt).

Nachteilig an den klassischen Verfahren der heterogen katalysierten Gasphasenoxidation von Propylen zu Acrolein und/oder Acrylsäure ist, daß sie einerseits als Propylenquelle im wesentlichen reines Propylen erfordern, Propylen jedoch ein im wesentlichen nicht natürlich vorkommender Stoff ist. In überwiegendem Ausmaß wird Propylen als Spaltgas beim Kracken von Erdöl-Kohlenwasserstoffen gewonnen. Andererseits vermag bei den klassischen Verfahren auch die Sauerstoffgrenzkonzentration des aus molekularem Sauerstoff, Propylen und inertem Verdünnungsgas bestehenden Beschickungsgases nicht zu befriedigen.

Unter der Sauerstoffgrenzkonzentration des Beschickungsgasgemisches versteht man denjenigen prozentualen Volumenanteil an molekularem Sauerstoff des Beschickungsgasgemisches, bei dessen Unterschreiten unabhängig von der Quantität der Volumenanteile der anderen Bestandteile des Beschickungsgasgemisches (diese Volumenanteile können im kontinuierlichen Betrieb infolge von Störungen in nicht beabsichtigter Weise fluktuieren), nämlich der partiell zu oxidierenden organischen Verbindung Propylen sowie dem inerten Verdünnungsgas, eine durch eine örtliche Zündquelle (wie z.B. lokale Überhitzung oder Funkenbildung im Reaktor) eingeleitete Verbrennung der organischen Substanz bei gegebenem Druck und Temperatur des Beschickungsgasgemisches sich in selbigem nicht mehr von der Zündquelle her auszubreiten vermag, so daß die Gefahr einer Explosion ausgeschlossen ist. Das heißt, aus Sicherheitsgründen muß der Volumenanteil des als Oxidationsmittel eingesetzten molekularen Sauerstoffs im Beschickungsgasgemisch unterhalb der sogenannten Sauerstoffgrenzkonzentration liegen. Da es andererseits bezüglich der Stöchiometrie der Partialoxidation zur gewünschten Zielverbindung in der Regel erforderlich ist, den als Oxidationsmittel verwendeten molekularen Sauerstoff in wenigstens stöchiometrischen oder in überstöchiometrischen Mengen einzusetzen (z.B. aus Gründen der Re-Oxidation der als Katalysator eingesetzten oxidischen Masse sowie zur Minderung von Kohlenstoffabscheidungen), beeinflußt die Sauerstoffgrenzkonzentration des Beschickungsgasgemisches den maximalen Volumenanteil des Beschickungsgemisches an der partiell zu oxidierenden organischen Verbindung (Propylen) und damit die erreichbare Raum-Zeit-Ausbeute an Zielprodukt (vgl. auch EP-A 257 565, Seite 5, Zeilen 36/37).

Da das Augenmerk des Durchschnittsfachmanns auf eine möglichst hohe Raum-Zeit-Ausbeute der gewünschten Zielverbindung gerichtet ist, besteht Interesse daran, den Volumenanteil der Reaktionspartner im Beschickungsgasgemisch möglichst hoch zu wählen, d.h. das inerte Verdünnungsgas so auszuwählen, daß es eine möglichst hohe Sauerstoffgrenzkonzentration bedingt.

Das G.B. Patent Nr. 1 450 986 empfiehlt, insbesondere aufgrund seiner erhöhten Wärmeaufnahmefähigkeit, die Verwendung von Kohlendioxid als im wesentlichen alleiniges inertes Verdünnungsgas zur Vermeidung der Explosionsgefahr bei der gasphasenkatalytisch oxidativen Herstellung von Acrylsäure aus Propylen.

Die DE-A 19 24 431 betrifft ebenfalls ein Verfahren der gasphasenkatalytisch oxidativen Herstellung von Acrylsäure aus Propylen. Als geeignete inerte Verdünnungsgase nennt die DE-A 19 62 431 Stickstoff, Wasserdampf, Kohlendioxid oder gesättigte Kohlenwasserstoffe.

DE DE-AS 22 51 364 empfiehlt für das Verfahren der heterogen katalysierten Gasphasen-Partialoxidation von Propylen zu Acrylsäure Wasserdampf als inertes Verdünnungsgas, dem Stickstoff oder gesättigte Kohlenwasserstoffe wie Methan, Propan oder Butan zugeführt werden können. Die DE-A 14 68 429 empfiehlt für ein Verfahren der heterogen katalysierten Gasphasenoxidation von Propylen zu Acrylsäure als inerte Verdünnungsgase Kohlendioxid, Stickstoff, gesättigte Kohlenwasserstoffe oder Wasserdampf, wobei Wasserdampf bevorzugt wird.

Sowohl die Gesamtheit der Ausführungsbeispiele der DE-A 19 62 431 als auch der DE-AS 22 51 364 und der DE-A 14 68 429 umfassen jedoch kein Beispiel, bei dem ein gesättigter Kohlenwasserstoff als inertes Verdünnungsgas auch nur mitverwendet worden wäre.

Die DE-A 30 06 894 betrifft ebenfalls die Problematik, bei einem heterogen katalysierten Gasphasen-Partialoxidationsverfahren des Propylens einerseits das Durchgehen der Reaktion auszuschließen und andererseits eine möglichst hohe Produktivität zu erzielen (Seite 2, Zeilen 11 bis 19) . Als Lösung empfiehlt sie, das Beschickungsgasgemisch bei geringerer Katalysatoraktivität zuzuführen und anschließend längs der Reaktionskoordinate die Katalysatoraktivität sukzessive zu erhöhen. Als mögliches inertes Verdünnungsgas benennt die DE-A 30 06 894 Stickstoff, Kohlendioxid und/oder Wasserdampf.

Die DT-AS 17 93 302 betrifft ein Verfahren der heterogen katalysierten Gasphasen-Partialoxidation, bei dem als inertes Verdünnungsgas nach Abtrennung des Zielproduktes das die in der Reaktion erzeugten Kohlenoxide und Wasserdampf enthaltende Reaktionsabgas verwendet wird. Die DE-A 20 56 614 spricht ebenfalls die Problematik des Ausschlusses explosionsartiger Verbrennungsprozesse bei der heterogen katalysierten Gasphasen-Partialoxidation von Propylen an (z.B. Seite 3, Absatz 2, letzte zwei Zeilen) . Um nachteilige Auswirkungen des bevorzugten Verdünnungsgases Wasserdampf zu vermeiden, empfiehlt die DE-A 20 56 614 die von kondensierbaren Gasen weitgehend befreiten Reaktionsabgase unter teilweisem oder vollständigem Ersatz des Wasserdampfes als inerte Verdünnungsgase in den Oxidationsreaktor rückzuführen und gleichzeitig das Beschickungsgasgemisch bei niederer Katalysatoraktivität zuzuführen und anschließend die Katalysatoraktivität längs der Reaktionskoordinate sukzessive zu erhöhen. Da das Oxidationsmittel "molekularer Sauerstoff" als Bestandteil von Luft zugeführt wird, sind die effektiven inerten Verdünnungsgase bei der Verfahrensweise der DE-A 20 56 614 im wesentlichen Stickstoff und Kohlendioxid. Die Verfahrensweise der DE-A 24 36 818 entspricht hinsichtlich der verwendeten inerten Verdünnungsgase im wesentlichen jener der DE-A 20 56 614. Das gleiche trifft auf die US-A 4,147,885 zu. Die DE-A 27 29 841 betrifft ein Verfahren der heterogen katalysierten Gasphasenoxidation von Propylen zu Acrylsäure, das aufgrund der Verwendung eines speziellen Oxidationskatalysators die Möglichkeit eröffnet, anstelle von Wasserdampf als inertes Verdünnungsmittel ein Gemisch aus CO, CO₂, Stickstoff und Argon, das aus dem Produktgasgemisch der heterogen katalysierten Partialoxidation abgetrennt und in das Beschickungsgasgemisch rückgeführt wird, zu verwenden.

Die EP-B 253 409 (vgl. insbesondere Seite 5, erste drei Zeilen) und die EP-A 257 565 lehren im Hinblick auf die Vermeidung eines Explosionsrisikos bei der heterogen katalysierten Gasphasen-Partialoxidation des Propylens die Verwendung von solchen inerten Verdünnungsgasen, die eine erhöhte molare Wärmekapazität Cp aufweisen. Als bevorzugt werden dabei z.B. auf Seite 4, Zeilen 47 ff. der EP-B 253 409 sowie auf Seite 5, Zeilen 26 ff. der EP-A 257 565 Gemische aus Stickstoff, CO₂, Methan, Ethan, Propan und Wasserdampf empfohlen. Neben den genannten Gasen können aber auch noch Helium, Argon, andere gesättigte Kohlenwasserstoffgase, N₂O und Kohlenmonoxid enthalten sein. Als für die Wirkung des inerten Verdünnungsgases wesentlich wird lediglich dessen mittlere molare Wärmekapazität erachtet. So besteht das inerte Verdünnungsgas des Beschickungsgasgemisches in allen Ausführungsbeispielen zu mehr als 55 Vol.-% aus N₂. Als besonders bevorzugt empfehlen die EP-B 253 409 (Seite 5, Zeile 41) und die EP-A 257 565 (Seite 6, Zeile 26) solche inerten Verdünnungsgasgemische, deren spezifische molare Wärme Cp unter den Betriebsbedingungen 10 bis 17 cal/mol K beträgt. Im wesentlichen reines Propan liegt mit einem entsprechenden Cp von 29,75 cal/mol K weit außerhalb dieser Empfehlung.

Die EP-A 293 224 betrifft ein Verfahren der heterogen katalysierten Gasphasenoxidation von Propylen zu Acrylsäure, bei dem zur Gewährleistung einer sicheren Verfahrensdurchführung die Verwendung eines Kohlendioxid, Wasserdampf und 1 bis 5 C-Atome aufweisende gesättigte Kohlenwasserstoffe enthaltenden Gasgemisches als Inertgas empfohlen wird (Seite 3, Zeilen 9 und 20 der EP-A 293 224) . Als wesentlich für die Wirksamkeit des seitens der EP-A 293 224 empfohlenen Inertgasgemisches erachtet die EP-A 293 224 das Beisein von Kohlenoxiden in erhöhten Konzentrationen (Seite 3, Zeile 57) sowie eine erhöhte molare Wärmekapazität des Inertgasgemisches (Seite 3, Zeile 57). Als weiteren besonderen Vorteil der ihrerseits empfohlenen Verfahrensweise erachtet die EP-A 293 224 die Tatsache, daß das zu verwendende Inertgasgemisch zu erheblichen Teilen aus dem Produktgasgemisch der Partialoxidation rekrutiert werden kann. In allen Ausführungsbeispielen umfaßt das im Beschickungsgasgemisch verwendete Inertgasgemisch Wasserdampf und CO₂ in einer Gesamtmenge von wenigstens 15 Vol.-%, bezogen auf das Inertgasgemisch.

Nachteilig an den vorstehend aufgeführten Verfahren der heterogen katalysierten Gasphasenoxidation von Propylen zu Acrylsäure des Standes der Technik ist, daß die damit einhergehenden Sauerstoffgrenzkonzentrationen nicht zu befriedigen vermögen und die Verfahren als Propylenquelle von im wesentlichen reinem Propylen ausgehen. Ersteres gilt ebenso für die Verfahrensweise der EP-A 117 146. Die EP-A 117 146 betrifft ein Verfahren zur Umsetzung von in Erdgas natürlich vorkommendem Propan zu Acrylsäure. Dabei wird in einer ersten Verfahrensstufe das Propan einer heterogen katalysierten partiellen Dehydrierung in der Gasphase zu Propylen unterworfen. Das dabei gebildete Propylen wird anschließend der heterogen katalysierten Gasphasen-Partialoxidation zu Acrylsäure unterworfen. Da die heterogen katalysierte Dehydrierung von Propan zu Propylen aus Gründen der Selektivität üblicherweise bei signifikant unter 100 % liegenden Propanumsätzen durchgeführt wird, kommt dieser Herstellungsvariante von Propylen normalerweise keine Bedeutung zu, erfordert doch das eng benachbarte Siedeverhalten von gebildetem Propylen und nicht umgesetztem Propan einen hohen Aufwand für die Trennung dieser Komponenten. Desweiteren ist eine Abtrennung von Nebenprodukten wie z.B. Wasserstoff erforderlich. Das erfindungswesentliche Merkmal der EP-A 117 146 besteht daher in der Entdeckung, daß sich die neben Propylen im Produktgasgemisch der Propandehydrierung befindlichen Hauptbestandteile bezüglich der nachfolgenden heterogen katalysierten Gasphasen-Partialoxidation des Propylens im wesentlichen inert verhalten, so daß man das Produktgasgemisch der Propandehydrierung ohne wesentliche Nachteile in seiner Gesamtheit in die nachfolgende Propylenoxidationsstufe überführen und die sich dabei inert verhaltenden Bestandteile anschließend in die Propandehydrierungsstufe rückführen kann. Anlaß, von dieser Verfahrensweise der EP-A 117 146 abzuweichen, bestand für den Fachmann auch angesichts der EP-B 253 409 und EP-A 257 565 nicht, liegt doch die mittlere molare spezifische Wärme eines aus Propan und Wasserstoff bestehenden Inertgasgemisches gerade im seitens der EP-A 117 146 und der EP-B 253 409 als bevorzugt empfohlenen Bereich. Vorstehend genannte Schriften legen allenfalls nahe, im Rahmen der Propandehydrierung mitverwendeten und/oder gebildeten Wasserdampf vor Weiterleitung des Dehydrierungsproduktgasgemisches auszufrieren. Wie bereits erwähnt, besteht der Nachteil der seitens der EP-A 117 146 empfohlenen Verfahrensweise jedoch darin, daß die Sauerstoffgrenzkonzentration des Beschickungsgasgemisches der Propylenoxidationsstufe auch im Rahmen dieser Verfahrensweise nicht zu befriedigen vermag.

Die Aufgabe der vorliegenden Erfindung bestand daher darin, ein Verfahren zur Herstellung von Acrolein, Acrylsäure oder deren Gemisch zur Verfügung zu stellen, das die Nachteile der Verfahren des Standes der Technik nicht aufweist.

Demgemäß wurde ein Verfahren zur Herstellung von Acrolein, Acrylsäure oder deren Gemisch aus Propan, bei dem man
A) in einer ersten Stufe A das Propan einer partiellen heterogen katalysierten Dehydrierung in der Gasphase zu Propylen unterwirft,
B) das Propylen und nicht umgesetztes Propan enthaltende Produktgasgemisch der Stufe A in einer zweiten Stufe B zur Beschickung eines Oxidationsreaktors verwendet und in dem Oxidationsreaktor das Propylen einer selektiven heterogen katalysierten Gasphasen-Partialoxidation mit molekularem Sauerstoff zu Acrolein, Acrylsäure oder deren Gemisch als Zielprodukt unterwirft, wobei als Sauerstoffquelle reiner Sauerstoff verwendet wird, und
C) aus dem im Rahmen der partiellen Oxidation des Propylens in der Stufe B anfallenden Produktgasstrom in einer dritten Stufe C das Zielprodukt abtrennt und wenigstens das im Produktgasstrom der Stufe B enthaltene nicht umgesetzte Propan in die Dehydrierungsstufe A zurückführt,
gefunden, das dadurch gekennzeichnet ist, daß man aus dem Produktgasgemisch der Stufe A vor seiner Verwendung zur Beschickung des Oxidationsreaktors der zweiten Stufe B von den darin enthaltenen, von Propan und Propylen verschiedenen, Bestandteilen wenigstens den Wasserstoff und den Wasserdampf abtrennt.

Das heißt, nach Zusatz von molekularem Sauerstoff wird der Oxidationsstufe B ein Beschickungsgasgemisch zugeführt, das im wesentlichen nur aus Propylen, molekularem Sauerstoff und Propan besteht. Letzterer Bestandteil bildet im wesentlichen das inerte Verdünnungsgas, während die ersten beiden Bestandteile die Reaktanten bilden. Die Vorteilhaftigkeit dieses Beschickungsgasgemisches ist zweifach begründet. Zum ersten ist es ausgehend von dem natürlich vorkommenden Rohstoff Propan in einfacher Weise erhältlich (Wasserstoff und Wasserdampf können in an sich bekannter einfacher Weise abgetrennt werden, wohingegen die aufwendige Propan/Propylen-Trennung unterbleiben kann) und zum anderen weist das Gemisch Sauerstoff/Propylen/Propan eine erhöhte Sauerstoffgrenzkonzentration auf. Letzterer Sachverhalt ist das Ergebnis ausführlicher und systematischer Forschungsarbeiten. Er fußt auf der Erkenntnis, daß für die Sauerstoffgrenzkonzentration eines aus molekularem Sauerstoff, inertem Verdünnungsgas und Propylen bestehenden Beschickungsgasgemisch (längs der Reaktionskoordinate nimmt der Anteil an molekularem Sauerstoff jeweils ab) weniger die molare Wärmekapazität Cp des Verdünnungsgases als vielmehr die Eigenschaft des inerten Verdünnungsgases, im wesentlichen selbst eine brennbare niedere organische Verbindung zu sein, von Relevanz ist, wobei das gleichzeitige Erfordernis der Inertheit die Beschränkung auf niedere gesättigte Kohlenwasserstoffe bedingt. Unter letzteren ist Propan insofern von Vorteil, als es bei nicht vollständig quantitativ inertem Verhalten in der Oxidationsstufe im wesentlichen selbst zu Propylen, Acrolein und/oder Acrylsäure umgesetzt wird.

Das Merkmal brennbar bringt dabei zum Ausdruck, daß es sich bei Propan um eine Verbindung handelt, deren bei einem Ausgangsdruck von 1 bar und einer Ausgangstemperatur von 50 bis 150°C befindliche Mischungen mit Luft eine obere und eine untere Explosionsgrenze (Entzündungsgrenze) aufweisen, wobei sich die Ermittlung der Explosionsgrenzen auf eine Bestimmung in der Standard Apparatur gemäß W. Berthold et al in Chem.-Ing. Tech. 56 (1984) Nr. 2, S. 126-127 bezieht.

Unter Explosionsgrenzen werden dabei nachfolgende Grenzwerte gemäß DIN 51649 verstanden:

In einem Gemisch aus Luft und einem brennbaren Gas ist die Geschwindigkeit, mit der sich unter vorgegebenen Ausgangsbedingungen eine durch eine örtliche Zündquelle (z.B. glühender Platindraht) eingeleitete Verbrennung (Entzündung, Explosion) ausbreitet, vom Gehalt an brennbarem Gas abhängig. Sie ist bei einem bestimmten Gehalt am größten. Sowohl bei Verringerung als auch bei Erhöhung des Gehaltes an brennbarem Gas wird die Verbrennungsgeschwindigkeit kleiner, bis sich schließlich die Verbrennungsreaktion bei einem unteren und einem oberen Grenzwert für den Gehalt an brennbarem Gas gerade nicht mehr von der Zündquelle her ausbreitet. Diese beiden Grenzwerte sind die untere Explosionsgrenze und die obere Explosionsgrenze, der dazwischen liegende Bereich des Gehaltes an brennbarem Gas ist der Explosionsbereich (Entzündungsbereich).

Je höher der Anteil an Propan im inerten Verdünnungsgas des Beschickungsgasgemisches der heterogen katalysierten Gasphasenoxidation von Propylen ist, desto sicherer läßt sich diese auch bei erhöhten Volumenanteilen der Reaktanten durchführen.

Um bei der Propandehydrierung interessante Umsätze zu erreichen, muß man bei relativ hohen Reaktionstemperaturen arbeiten (in typischer Weise 300 bis 700°C). Da die Dehydrierung (Spaltung von C-H) gegenüber der Crackung (Spaltung von C-C) thermodynamisch benachteiligt ist, erfolgt sie an selektiv wirkenden Katalysatoren. Pro gebildetem Propylenmolekül wird ein Wasserstoffmolekül als Nebenprodukt erzeugt. Infolge der selektiv wirkenden Katalysatoren entstehen Methan und Ethan als weitere Nebenprodukte nur in untergeordneten Mengen. Da die Dehydrierreaktion unter Volumenzunahme abläuft, kann der Umsatz durch Erniedrigung des Partialdrucks der Reaktionspartner gesteigert werden. Dies läßt sich in einfacher Weise z.B. durch Zumischen von Wasserdampf erreichen, der für die eigentliche Dehydrierreaktion ein Inertgas darstellt. Eine Verdünnung mit Wasserdampf bedingt als weiteren Vorteil ein vermindertes Verkoken des verwendeten Katalysators, da der Wasserdampf mit diesem nach der Wassergasreaktion reagiert. Außerdem läßt sich Wasserdampf aus dem Produktgasgemisch der Dehydrierung z.B. durch Kondensieren leicht abtrennen. Ein weiterer Weg zur Umsatzsteigerung der Dehydrierung ist die Herausnahme von Wasserstoff aus dem Gleichgewicht auf chemischem Wege. Als einfachste Möglichkeit bietet sich das Hinzufügen von Sauerstoff zum Reaktionsgemisch an. Man spricht dann von oxidativer Dehydrierung, bei der als Nebenprodukt infolge der Umsetzung des Sauerstoffs mit Wasserstoff Wasserdampf als Nebenprodukt gebildet wird. Die Zusammenhänge sind dem Fachmann im Detail bekannt und z.B. in den nachfolgenden Schriften einschließlich zu verwendender Dehydrierkatalysatoren beschrieben: EP-A 117 146, US-A 3,784,483, US-A 4,083,883, US-A 3,692,701, US-A 4,005,985, US-A 4,041,099, US-A 4,144,277 und US-A 4,176,410. Als Produktgasgemisch können daher solche anfallen, deren Hauptbestandteile nicht umgesetztes Propan, gegebenenfalls nicht umgesetzter Sauerstoff, als Zielprodukt gebildetes Propylen, als Nebenprodukt gebildeter Wasserstoff sowie gegebenenfalls Wasserdampf bilden. Höchstens in untergeordneten Mengen sind Methan, Ethan, CO und CO₂ enthalten.

Das heißt, nach Abtrennung des im Dehydrierproduktgasgemisch enthaltenen Wasserstoffes und Wasserdampfes (z.B. durch fraktionierte Kondensation) wird ein Gasgemisch erhalten, das im wesentlichen nur noch aus Propylen, Propan und gegebenenfalls geringen Mengen an O₂ besteht und im Rahmen des erfindungsgemäßen Verfahrens zur Beschickung des Oxidationsreaktors der Stufe B verwendet werden kann. Die dem Beschickungsgasgemisch zuzumischende Menge des als Oxidationsmittel wirkenden molekularen O₂ wird an die darin enthaltene Propylenmenge angepaßt. Das Propylen/Propan-Verhältnis wird in dem Fachmann bekannter Weise durch den Umsatz der Dehydrierung eingestellt. Als Ergebnis der vorstehend beschriebenen Vorgehensweise sollte das Beschickungsgasgemisch des Oxidationsreaktors der Stufe B im Rahmen des erfindungsgemäßen Verfahrens, bezogen auf darin enthaltenes Propan, nicht mehr als 5 Vol.-% an von Propan, Propylen, Ethan, Methan und molekularem Sauerstoff verschiedenen Bestandteilen enthalten. In einer bevorzugten Ausführungsform des erfindungsgemäßen Verfahrens werden aus dem Produktgasgemisch der Stufe A des erfindungsgemäßen Verfahrens alle von Propan, Propylen und gegebenenfalls molekularem Sauerstoff verschiedenen Bestandteile abgetrennt. Die dabei anzuwendenden Trennverfahren wie fraktionierte Kondensation oder Absorptions-, Extraktionsverfahren sind dem Fachmann bekannt und bedürfen keiner näheren Erläuterung. CO₂ kann beispielsweise durch Waschen des Gasgemisches in wäßriger basischer Lösung abgetrennt werden. Da molekularer Sauerstoff in Luft nur mit N₂ vergesellschaftet vorkommt, ist der für die Oxidationsstufe B des erfindungsgemäßen Verfahrens als Oxidationsmittel benötigte molekulare Sauerstoff erfindungsgemäß einer im wesentlichen reinen Sauerstoffquelle zu entnehmen.

Wie bereits erwähnt, läuft die heterogen katalysierte Gasphasen-Partialoxidation von Propylen zu Acrylsäure mit molekularem Sauerstoff prinzipiell in zwei längs der Reaktionskoordinate aufeinanderfolgenden Schritten ab, von denen der erste zum Acrolein und der zweite vom Acrolein zur Acrylsäure führt. Dieser Reaktionsablauf in zwei zeitlich aufeinanderfolgenden Schritten eröffnet in an sich bekannter Weise die Möglichkeit, die Stufe B des erfindungsgemäßen Verfahrens in Gestalt zweier hintereinander angeordneten Oxidationsstufen auszuführen, wobei in jeder der beiden Oxidationsstufen der zu verwendende oxidische Katalysator in optimierender Weise angepaßt werden kann. So wird für die erste Oxidationsstufe (Propylen → Acrolein) in der Regel ein Katalysator auf der Basis von die Elementkombination Mo-Bi-Fe enthaltenden Multimetalloxiden bevorzugt, während für die zweite Oxidationsstufe (Acrolein → Acrylsäure) normalerweise Katalysatoren auf der Basis von die Elementkombination Mo-V enthaltenden Multimetalloxiden bevorzugt werden. Entsprechende Multimetalloxidkatalysatoren für die beiden Oxidationsstufen sind vielfach vorbeschrieben und dem Fachmann wohlbekannt. Beispielsweise verweist die EP-A 253 409 auf Seite 5 auf entsprechende US-Patente. Günstige Katalysatoren für die beiden Oxidationsstufen offenbaren auch die DE-A 44 31 957 und die DE-A 44 31 949, dies gilt insbesondere für jene der allgemeinen Formel I in den beiden Schriften. In der Regel wird das Produktgemisch der ersten Oxidationsstufe ohne Zwischenbehandlung in die zweite Oxidationsstufe überführt. Die einfachste Realisierungsform der beiden Oxidationsstufen bildet daher ein Rohrbündelreaktor innerhalb dessen sich die Katalysatorbeschickung längs der einzelnen Kontaktrohre mit Beendigung des ersten Reaktionsschrittes entsprechend ändert. Vorzugsweise werden die beiden Oxidationsstufen jedoch in Form eines aus zwei hintereinander geschalteten Oxidationsreaktoren bestehenden Oxidationsreaktors realisiert. In diesem Fall können auch die übrigen Reaktionsbedingungen, z.B. die Reaktionstemperatur, in der jeweiligen Oxidationsstufe in einfacher Weise optimierend angepaßt werden. Zweckmäßigerweise führt man in diesem Fall den für die zweite Oxidationsstufe benötigten molekularen Sauerstoff erst dem Beschickungsgasgemisch des zweiten Oxidationsreaktors zu. Vorzugsweise wird dabei für beide Oxidationsstufen im Beschickungsgasgemisch eine partiell zu oxidierende organische Verbindung (Propylen bzw. Acrolein) : molekularer Sauerstoff Beschickung im Volumenverhältnis 1 : 1 bis 3, vorzugsweise 1 : 1,5 bis 2 gewählt (dies gilt auch für eine Stufe B, die bei Acrolein endet). Wie bereits erwähnt, wirkt sich der Sauerstoffüberschuß in beiden Oxidationsstufen vorteilhaft auf die Kinetik der Gasphasenoxidation aus. Die thermodynamischen Verhältnisse werden dadurch im wesentlichen nicht beeinflußt, da die heterogen katalysierte Gasphasen-Partialoxidation des Propylens zu Acrylsäure kinetischer Kontrolle unterliegt. Prinzipiell ist die heterogen katalysierte Gasphasen-Partialoxidation von Propylen zu Acrylsäure aber auch einstufig realisierbar. In diesem Fall erfolgen beide Reaktionsschritte in einem Oxidationsreaktor der mit einem Katalysator beschickt ist, der die Umsetzung beider Reaktionsschritte katalysiert. Selbstverständlich kann sich auch die Katalysatorbeschickung innerhalb einer Oxidationsstufe längs der Reaktionskoordinate kontinuierlich oder abrupt ändern. Natürlich kann bei einer Ausführungsform der erfindungsgemäßen Stufe B in Gestalt zweier hintereinandergeschalteter Oxidationsreaktoren aus dem das den ersten Oxidationsreaktor verlassenden Produktgasgemisch in selbigem enthaltenes, im ersten Oxidationsreaktor als Nebenprodukt entstandenes, Kohlenoxid und Wasserdampf bei Bedarf vor Weiterleitung in den zweiten Oxidationsreaktor abgetrennt werden.

Im übrigen sind dem Fachmann die in der Stufe B zu wählenden Reaktionstemperaturen und Drücke aus der Literatur bekannt.

Das die Stufe B verlassende Produktgasgemisch ist im wesentlichen zusammengesetzt aus dem Zielprodukt Acrolein, Acrylsäure oder deren Gemisch, nicht umgesetztem Propylen, nicht umgesetztem Acrolein, nicht umgesetztem molekularem Sauerstoff, als Nebenprodukt entstandenem Wasserdampf, als Nebenprodukt entstandenen Kohlenoxiden, dem inerten Verdünnungsgas Propan sowie geringen Mengen sonstiger niederer Aldehyde und Kohlenwasserstoffe.

Das Zielprodukt wird in an sich bekannter Weise aus dem Produktgasgemisch abgetrennt (z.B. Absorption von Acrylsäure in Wasser oder in einem hochsiedenden hydrophoben organischen Lösungsmittel oder Absorption von Acrolein in Wasser oder in wäßrigen Lösungen niederer Carbonsäuren sowie anschließende Aufarbeitung der Absorbate; vgl. z.B. EP-A 117 146 und DE-A 43 08 087 oder DE-A 43 35 172 sowie DE-A 44 36 243). Nicht umgesetztes Propylen und/oder Acrolein werden gegebenenfalls gleichfalls abgetrennt und in die Stufe B rückgeführt. Ansonsten können die von Acrylsäure und Acrolein verschiedenen wesentlichen Bestandteile je nach Bedarf und verwendetem Dehydrierkatalysator jeweils für sich abgetrennt oder zusammen mit dem Propan in die Dehydrierstufe A rückgeführt werden, um dort, wie beschrieben, den Dehydrierungsumsatz zu beeinflussen. Selbstverständlich kann aber auch Propan für sich in die Stufe A rückgeführt werden. Bei kontinuierlicher Ausführung des erfindungsgemäßen Verfahrens erfolgt so eine kontinuierliche Umsetzung von Propan zu Acrylsäure und/oder Acrolein.

Die Unterschiede des erfindungsgemäßen Verfahrens zu den Verfahren des Standes der Technik bestehen zusammenfassend im wesentlichen darin, daß einmal von Propan als Ausgangsstoff ausgegangen werden kann und bei Anwendung von ansonsten identischen Reaktionsbedingungen infolge der Funktion von nicht umgesetztem Propan als im wesentlichen alleinigem inertem Verdünnungsgas die heterogen katalysierte Gasphasenoxidation von Propylen zu Acrolein, Acrylsäure oder deren Gemisch, insbesondere bei erhöhten Reaktantenvolumenanteilen im Beschickungsgasgemisch der Gasphasenoxidation, mit erhöhter Sicherheit durchführbar ist (auch die Beschickung mit molekularem Sauerstoff kann im kontinuierlichen Betrieb aufgrund von unbeabsichtigten Störungen fluktuieren), was die Grundlage für erhöhte Raum-Zeit-Ausbeuten an Zielprodukt bildet. Nachfolgend wird dies in einigen Ausführungsbeispielen ausgewiesen. Es sei festgehalten, daß gemäß der erfindungsgemäßen Verfahrensweise Beschickungsgasgemische der Stufe B sicherer handhabbar sind, deren Propylenbeschickung >30 Vol.-% bis zu 40 bis 45 Vol.-%, bezogen auf das Beschickungsgasgemisch, beträgt.

Günstige Beschickungsgasgemische der Stufe B umfassen
15 bis 30 Vol.-% Propylen
20 bis 40 Vol.-% Sauerstoff und
30 bis 65 Vol.-% Propan.

Sie sind in einfacher Weise dadurch erhältlich, daß man den Umsatz in der Stufe A zu 25 bis 35 mol-% wählt und nach Abtrennung von Wasserstoff, Wasserdampf und gegebenenfalls sonstigen Nebenprodukten die entsprechende Menge an molekularem Sauerstoff zusetzt.

In besonders vorteilhafter Weise läßt sich das erfindungsgemäße Verfahren anwenden, wenn als Rohstoff zur gasphasenkatalytisch oxidativen Herstellung von Acrolein und/oder Acrylsäure von Raffineriepropylen ausgegangen wird. Selbiges besteht zu etwa 70 Vol.-% aus Propylen und zu etwa 30 Vol.-% aus Propan.

Zweckmäßigerweise wird man das Raffineriepropylen zunächst zur Beschickung eines Oxidationsreaktors verwenden und in dem Oxidationsreaktor das Propylen gemäß Stufe B des erfindungsgemäßen Verfahrens einer selektiven heterogen katalysierten Gasphasen-Partialoxidation zu Acrolein, Acrylsäure oder deren Gemisch als Zielprodukt unterwerfen und aus dem im Rahmen der partiellen Oxidation des Propylens in der Stufe B anfallenden Produktstrom in einer erfindungsgemäßen Stufe C) das Zielprodukt abtrennen und wenigstens das im Produktgasstrom der Stufe B enthaltene nicht umgesetzte Propan in eine erfindungsgemäße Dehydrierstufe A zurückführen, um anschließend in erfindungsgemäßer Weise weiterzuverfahren.

### Beispiele (Einfluß der Brennbarkeit der inerten Verdünnungsgasbestandteile auf die Sauerstoffgrenzkonzentration)

Ermittlung der Sauerstoffgrenzkonzentration von bei einer Ausgangstemperatur von 250°C und einem Ausgangsdruck von 1 bar befindlichen Beschickungsgasgemischen, bestehend aus Propylen (partiell zu oxidierende organische Verbindung), molekularem Sauerstoff (Oxidationsmittel) und einem bezüglich einer heterogen katalysierten Gasphasen-Partialoxidation des Propylens zu Acrylsäure inerten Verdünnungsgas.

### Allgemeine Versuchsdurchführung:

Die Versuche wurden in einem geschlossenen kugelförmigen 5l-Hochdruckbehälter aus Edelstahl durchgeführt. Die Erzeugung des Gasgemisches in dem anfangs evakuierten Hochdruckbehälter erfolgte nach der Partialdruckmethode. Nach einer Mischzeit von 10 min mittels eines Magnetrührwerkes wurde versucht, das Gasgemisch mittels eines durchschmelzenden Platindrahtes zu zünden. Eine dadurch eventuell ausgelöste selbständige Ausbreitung einer Reaktionsfront (Explosion) wurde über den zeitlichen Anstieg des Behälterinnendruckes (Messung mit piezoelektrischem Druckaufnehmer) und über die Erhöhung der Temperatur im Behälter detektiert.

Ergebnisse (die zugrunde gelegten spezifischen molaren Wärmen Cp fußen auf den Daten aus "Ihsan Barin, Thermochemical Data of Pure Substances, Part I u. Part II, VCH Verlagsgesellschaft, Weinheim, Zweite Auflage, 1993", wobei bei Gasgemischen ideales Gasverhalten angenommen wurde):
a) Ausschließliche Verwendung von Methan als inertes brennbares Verdünnungsgas. D.h. das inerte Verdünnungsgas bestand ausschließlich aus brennbaren Bestandteilen. Die spezifische molare Wärme Cp von Methan beträgt unter den vorgegebenen Bedingungen 47,5 J/mol·K. Die ermittelte Sauerstoffgrenzkonzentration beträgt 32 Vol.-%.
   D.h. in einem Gemisch aus Propylen, molekularem O₂ und Methan als Inertgas, das sich bei 250°C und 1 bar befindet, vermag sich eine lokale Entzündung (Explosion) unabhängig von der speziellen Gemischzusammensetzung dann nicht mehr selbständig auszubreiten, wenn der Volumenanteil an O₂ im Gesamtgemisch < 32 Vol.-% beträgt. D.h. in einem bei 1 bar und 250°C befindlichen Gemisch aus 31 Vol.-% O₂, 20 Vol.-% Propylen und 49 Vol.-% Methan vermag sich eine lokale Entzündung nicht mehr selbständig auszubreiten.
b) Verwendung eines 3,2 (Propan) : 96,8 (CO₂)-Gemisches (Verhältnis der Volumenanteile) aus Propan und Kohlendioxid als inertes Verdünnungsgas. D.h. das inerte Verdünnungsgas bestand fast ausschließlich aus nicht brennbarem Verdünnungsgas. Die Zusammensetzung des Inertgasgemisches wurde so gewählt, damit es unter den vorgegebenen Bedingungen ebenfalls ein Cp von 47,5 J/mol·K aufweist. Die ermittelte Sauerstoffgrenzkonzentration beträgt lediglich 15 Vol.-%.
   D.h. in einem unter entsprechenden Bedingungen wie in a) befindlichen Gemisch aus 31 Vol.-% O₂, 20 Vol.-% Propylen und 49 Vol.-% des inerten Verdünnungsgases breitet sich eine lokale Entzündung selbständig aus.
c) Verwendung eines 48,3 (Propan) : 51,7 (Methan)-Gemisches (Verhältnis der Volumenanteile) aus Propan und Methan als inertes Verdünnungsgas. D.h. das inerte Verdünnungsgas bestand ausschließlich aus brennbaren Bestandteilen. Die spezifische molare Wärme Cp dieses Gemisches beträgt unter den vorgegebenen Bedingungen = 80,8 J/mol·K. Die ermittelte Sauerstoffgrenzkonzentration beträgt 37 Vol.-%.
d) Verwendung eines 50 (Propan) : 50 (CO₂)-Gemisches (Verhältnis der Volumenanteile) aus Propan und Kohlendioxid als inertes Verdünnungsgas. D.h. das inerte Verdünnungsgas enthielt auch nicht brennbare Bestandteile. Die Zusammensetzung des Inertgasgemisches wurde so gewählt, damit es unter den vorgegebenen Bedingungen ebenfalls ein Cp von 80,8 J/mol·K aufweist.
   Die ermittelte Sauerstoffgrenzkonzentration beträgt lediglich 34 Vol.-%. D.h. trotz signifikant höherem Cp-Wert des inerten Verdünnungsgases im Vergleich zu a), liegt die Sauerstoffgrenzkonzentration nur bei einem vergleichbaren Volumenanteil wie bei a).

## Patentansprüche

1. Verfahren zur Herstellung von Acrolein, Acrylsäure oder deren Gemisch aus Propan, bei dem man
A) in einer ersten Stufe A das Propan einer partiellen heterogen katalysierten Dehydrierung in der Gasphase zu Propylen unterwirft,
B) das Propylen und nicht umgesetztes Propan enthaltende Produktgasgemisch der Stufe A in einer zweiten Stufe B zur Beschickung eines Oxidationsreaktors verwendet und in dem Oxidationsreaktor das Propylen einer selektiven heterogen katalysierten Gasphasen-Partialoxidation mit molekularem Sauerstoff zu Acrolein, Acrylsäure oder deren Gemisch als Zielprodukt unterwirft, wobei als Sauerstoffquelle reiner Sauerstoff verwendet wird, und
C) aus dem im Rahmen der partiellen Oxidation des Propylens in der Stufe B anfallenden Produktgasstrom in einer dritten Stufe C das Zielprodukt abtrennt und wenigstens das im Produktgasstrom der Stufe B enthaltene nicht umgesetzte Propan in die Dehydrierungsstufe A zurückführt,
dadurch gekennzeichnet, daß man aus dem Produktgasgemisch der Stufe A vor seiner Verwendung zur Beschickung des Oxidationsreaktors der zweiten Stufe B von den darin enthaltenen, von Propan und Propylen verschiedenen, Bestandteilen wenigstens den Wasserstoff und den Wasserdampf abtrennt.

2. Verfahren nach Anspruch 1, dadurch gekennzeichnet, daß man aus dem Produktgasgemisch der Stufe A vor seiner Verwendung zur Beschickung des Oxidationsreaktors der zweiten Stufe B die Gesamtmenge der darin enthaltenen, von Propan und Propylen verschiedenen, Bestandteile abtrennt.

3. Verfahren nach Anspruch 1 oder 2, dadurch gekennzeichnet, daß man die Stufe B in Gestalt zweier hintereinander geschalteter Oxidationsreaktoren realisiert, von denen der erste mit einem die Elementkombination Mo-Bi-Fe enthaltenden Multimetalloxidkatalysator und der zweite mit einem die Elementkombination Mo-V enthaltenden Multimetalloxidkatalysator beschickt ist.

4. Verfahren nach Anspruch 3, dadurch gekennzeichnet, daß man das Produktgasgemisch des ersten Oxidationsreaktors ohne Zwischenbehandlung in den zweiten Oxidationsreaktor überführt, und dabei in den Beschickungsgasgemischen beider Oxidationsreaktoren eine partiell zu oxidierende organische Verbindung (Propylen bzw. Acrolein) : zu molekularer Sauerstoff Beschickung im Volumenverhältnis 1 : 1 bis 3 einstellt.

## Claims

1. A process for preparing acrolein, acrylic acid or a mixture thereof from propane, in which
A) in a first stage A, the propane is subjected to a partial heterogeneously catalyzed dehydrogenation in the gas phase to give propylene,
B) the product gas mixture from stage A containing propylene and unreacted propane is used in a second stage B as feed to an oxidation reactor and in the oxidation reactor the propylene is subjected to a selective heterogeneously catalyzed gas-phase partial oxidation with molecular oxygen to give acrolein, acrylic acid or a mixture thereof as target product, with pure oxygen being used as oxygen source, and
C) in a third stage C, the target product is separated from the product gas stream obtained from the partial oxidation of the propylene in stage B and at least the unreacted propane present in the product gas stream from stage B is recirculated to the dehydrogenation stage A,
wherein, from among the constituents other than propane and propylene present in the product gas mixture from stage A, at least the hydrogen and the water vapor are separated from the product gas mixture before it is used as feed to the oxidation reactor of the second stage B.

2. A process as claimed in claim 1, wherein the total amount of constituents other than propane and propylene present in the product gas mixture of stage A are removed therefrom before it is used as feed to the oxidation reactor of the second stage B.

3. A process as claimed in claim 1 or 2, wherein the stage B is implemented in the form of two oxidation reactors connected in series, of which the first is charged with a multimetal oxide catalyst containing the element combination Mo-Bi-Fe and the second is charged with a multimetal oxide catalyst containing the element combination Mo-V.

4. A process as claimed in claim 3, wherein the product gas mixture of the first oxidation reactor is transferred without intermediate treatment to the second oxidation reactor, with the volume ratio of organic compound to be partially oxidized (propylene or acrolein):molecular oxygen being set at 1:1 to 3 in the feed gas mixtures of both oxidation reactors.

## Revendications

1. Procédé de préparation d'acroléine, d'acide acrylique ou de leurs mélanges à partir de propane, dans lequel
A) dans une première étape A, on soumet le propane à une déshydrogénation partielle catalysée de façon hétérogène en phase gaz pour donner du propylène,
B) dans une deuxième étape B, on alimente un réacteur d'oxydation avec le mélange de produit gazeux contenant le propylène et le propane non réagi de l'étape A, et on soumet, dans ce réacteur d'oxydation, le propylène à une oxydation partielle sélective catalysée de façon hétérogène, en phase gaz, avec de l'oxygène moléculaire pour donner de l'acroléine, de l'acide acrylique ou leurs mélanges, en tant que produit cible, la source d'oxygène utilisée étant de l'oxygène pur, et
C) dans une troisième étape C, on sépare le produit cible du courant de produits gazeux obtenu à l'issue de l'étape B après l'oxydation partielle du propylène, on renvoie dans l'étape de déshydrogénation A au moins le propane non réagi contenu dans le courant de produits gazeux de l'étape B,
caractérisé en ce que l'on sépare des composants distincts du propane et du propylène, au moins l'hydrogène et la vapeur d'eau, du mélange de produit gazeux de l'étape A avant son utilisation pour alimenter le réacteur d'oxydation de la deuxième étape B.

2. Procédé selon la revendication 1, caractérisé en ce que l'on sépare la totalité des composants distincts du propane et du propylène du mélange de produit gazeux de l'étape A avant son utilisation pour alimenter le réacteur d'oxydation de la deuxième étape B.

3. Procédé selon la revendication 1 ou 2, caractérisé en ce que l'on réalise l'étape B sous la forme de deux réacteurs d'oxydation connectés l'un à la suite de l'autre, le premier étant alimenté avec un catalyseur à oxyde multimétallique contenant la combinaison d'éléments Mo-Bi-Fe et le second étant alimenté avec un catalyseur à oxyde multimétallique contenant la combinaison d'éléments Mo-V.

4. Procédé selon la revendication 3, caractérisé en ce que l'on envoie le mélange de produits gazeux du premier réacteur d'oxydation dans le second réacteur d'oxydation sans traitement intermédiaire, et en ce qu'on règle dans les mélanges gazeux d'alimentation des deux réacteurs d'oxydation, un rapport en volume du composé organique à oxyder partiellement (propylène ou acroléine) à l'alimentation en oxygène moléculaire de 1:1 à 3.
